# EUROPEAN PATENT SPECIFICATION

(11) **EP 2 126 565 B1**
(45) Date of publication and mention of the grant of the patent: **26.10.2011**
(21) Application number: 08716004.0
(22) Date of filing: 25.02.2008
(51) Int. Cl.: G01N 33/50, G01N 33/74

(54) **METHOD FOR THE ASSESSMENT OF THE INHIBITORY ACTIVITY OF ANTIBODIES AGAINST INSULIN-LIKE GROWTH FACTOR I RECEPTOR**
VERFAHREN ZUR BEURTEILUNG DER HEMMUNGSAKTIVITÄT VON ANTIKÖRPERN GEGEN INSULINARTIGEN WACHSTUMSFAKTOR-1-REZEPTOR
PROCEDE POUR L'EVALUATION DE L'ACTIVITE INHIBITRICE D'ANTICORPS A L'ENCONTRE DU RECEPTEUR DU FACTEUR DE CROISSANCE I DE TYPE INSULINE

(30) Priority: 27.02.2007 EP 07003948
(43) Date of publication of application: 02.12.2009
(73) Proprietor: F. Hoffmann-La Roche AG, 4070 Basel (CH)
(72) Inventor: EVERS, Stefan, 79379 Muellheim (DE); HINZ, Andreas, 82377 Penzberg (DE); KUENKELE, Klaus-Peter, 83671 Benediktbeuern (DE)
(74) Representative: Burger, Alexander
(86) International application number: PCT/EP2008/001457
(87) International publication number: WO 2008/104344

(56) References cited:
- WO-A-02/053596
- WO-A-2004/087756
- WO-A-2005/005635
- COHEN B ET AL: "Combination therapy enhances the inhibition of tumor growth with the fully human anti-type 1 insulin-like growth factor receptor monoclonal antibody CP-751,871" CLINICAL CANCER RESEARCH, THE AMERICAN ASSOCIATION FOR CANCER RESEARCH, US, vol. 11, no. 5, 1 March 2005 (2005-03-01), pages 2063-2073, XP002433667 ISSN: 1078-0432 cited in the application
- KOOIJMAN RON ET AL: "Differential expression of type I insulin-like growth factor receptors in different stages of human T cells" EUROPEAN JOURNAL OF IMMUNOLOGY, vol. 25, no. 4, 1995, pages 931-935, XP002478574 ISSN: 0014-2980
- DOUGLAS RAYMOND S ET AL: "Aberrant expression of the insulin-like growth factor-1 receptor by T cells from patients with Graves' disease may carry functional consequences for disease pathogenesis" JOURNAL OF IMMUNOLOGY, vol. 178, no. 5, March 2007 (2007-03), pages 3281-3287, XP002478630 ISSN: 0022-1767

## Description

The present invention relates to methods for the assessment of the inhibitory activity of antibodies against insulin-like growth factor I receptor (IGF-1R), methods for predicting the pharmacokinetic and pharmacodynamic properties of such antibodies and methods for the prediction of a clinical outcome of a treatment course in a patient wherein such an antibody is administered.

Insulin-like growth factor I receptor (IGF-1R, EC 2.7.112, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1093). IGF-1R binds IGF-1 with high affinity and initiates the physiological response to this ligand in vivo. IGF-1R also binds to IGF-2, however with slightly lower affinity. IGF-1R overexpression promotes the neoplastic transformation of cells and there exists evidence that IGF-1R is involved in malignant transformation of cells and is therefore a useful target for the development of therapeutic agents for the treatment of cancer (Adams, T.E., et al., Cell. Mol. Life Sci. 57 (2000) 1050-1093). Insulin-like growth factor I (IGF I) is an important mitogen for vascular smooth muscle cells (VSMC). The IGF I receptor is a heterotetramer composed of two cross-linked extracellular alpha-chains and two membrane-spanning beta-chains that contain a tyrosine-kinase domain. It has a high degree of sequence similarity to the insulin receptor (IR), and the putative ligand-specific binding site has been localized to a cysteine-rich region (CRR) of the alpha-chain (Adams, T. E., et al., Cell. Mol. Life Sci 57 (2000) 1050-1093).

Antibodies against IGF-1R are well-known in the state of the art and investigated for their antitumor effects in vitro and in vivo (Benini, S., et al., Clin. Cancer Res. 7 (2001) 1790-1797; Scotlandi, K., et al., Cancer Gene Ther. 9 (2002) 296-307; Scotlandi, K., et al., Int. J. Cancer 101 (2002) 11-16; Brunetti, A., et al., Biochem. Biophys. Res. Commun. 165 (1989) 212-218; Prigent, S.A., et al., J. Biol. Chem. 265 (1990) 9970-9977; Li, S.L., et al., Cancer Immunol. Immunother. 49 (2000) 243-252; Pessino, A., et al., Biochem. Biophys. Res. Commun. 162 (1989) 1236-1243; Surinya, K.H., et al., J. Biol. Chem. 277 (2002) 16718-16725; Soos, M.A., et al., J. Biol. Chem., 267 (1992) 12955-12963; Soos, M.A., et al., Proc. Natl. Acad. Sci. USA 86 (1989) 5217-5221; O'Brien, R.M., et al., EMBO J. 6 (1987) 4003-4010; Taylor, R., et al., Biochem. J. 242 (1987) 123-129; Soos, M.A., et al., Biochem. J. 235 (1986) 199-208; Li, S.L., et al., Biochem. Biophys. Res. Commun. 196 (1993) 92-98; Delafontaine, P., et al., J. Mol. Cell. Cardiol. 26 (1994) 1659-1673; Kull, F.C. Jr., et al., J. Biol. Chem. 258 (1983) 6561-6566; Morgan, D.O., and Roth, R.A., Biochemistry 25 (1986) 1364-1371; Forsayeth, J.R., et al., Proc. Natl. Acad. Sci. USA 84 (1987) 3448-3451; Schaefer, E.M., et al., J. Biol. Chem. 265 (1990) 13248-13253; Gustafson, T.A., and Rutter, W.J., J. Biol. Chem. 265 (1990) 18663-18667; Hoyne, P.A., et al., FEBS Lett. 469 (2000) 57-60; Tulloch, P.A., et al., J. Struct. Biol. 125 (1999) 11-18; Rohlik, Q.T., et al., Biochem. Biophys. Res. Comm. 149 (1987) 276-281; and Kalebic, T., et al., Cancer Res. 54 (1994) 5531-5534; Adams, T. E., et al., Cell. Mol. Life Sci. 57 (2000) 1050-1093; Dricu, A., et al., Glycobiology 9 (1999) 571-579; Kanter-Lewensohn, L., et al., Melanoma Res. 8 (1998) 389-397; Li, S.L., et al., Cancer Immunol. Immunother. 49 (2000) 243-252). Antibodies against IGF-1R are also described in a lot of further publications, e.g., Arteaga, C.L., et al., Breast Cancer Res. Treatment 22 (1992) 101-106; and Hailey, J., et al., Mol. Cancer Ther. 1 (2002) 1349-1353.

In particular, the monoclonal antibody against IGF-1R called αIR3 is widely used in the investigation of studying IGF-1R mediated processes and IGF-1 mediated diseases such as cancer. Alpha-IR-3 was described by Kull, F.C., J. Biol. Chem. 258 (1983) 6561-6566. In the meantime, about a hundred publications have been published dealing with the investigation and therapeutic use of αIR3 in regard to its antitumor effect, alone and together with cytostatic agents such as doxorubicin and vincristine. αIR3 is a murine monoclonal antibody which is known to inhibit IGF-1 binding to IGF receptor but not IGF-2 binding to IGF-1R. αIR3 stimulates at high concentrations tumor cell proliferation and IGF-1R phosphorylation (Bergmann, U., et al., Cancer Res. 55 (1995) 2007-2011; Kato, H., et al., J. Biol. Chem. 268 (1993) 2655-2661). There exist other antibodies (e.g., 1H7, Li, S.L., et al., Cancer Immunol. Immunother. 49 (2000) 243-252) which inhibit IGF-2 binding to IGF-1R more potently than IGF-1 binding. A summary of the state of the art of antibodies and their properties and characteristics is described by Adams, T.E., et al., Cell. Mol. Life Sci. 57 (2000) 1050-1093.

Most of the antibodies described in the state of the art are of mouse origin. Such antibodies are, as is well known in the state of the art, not useful for the therapy of human patients without further alterations like chimerization or humanization. Based on these drawbacks, human antibodies are clearly preferred as therapeutic agents in the treatment of human patients. Human antibodies are well-known in the state of the art (van Dijk, M.A., and van de Winkel, J.G., Curr. Opin. Pharmacol. 5 (2001) 368-374). Based on such technology, human antibodies against a great variety of targets can be produced. Chimeric, humanized and human therapeutic antibodies against IGF-1R are mentioned in WO 02/053596, WO 2004/071529, WO 2005/016967 WO 2006/008639, US 2005/0249730, US 2005/0084906, WO 2005/058967, WO 2006/013472, US 2003/0165502, WO 2005/082415, WO 2005/016970, WO 03/106621, WO 04/083248, WO 2003/100008, WO 2004/087756, WO 2005/005635 and WO 2005/094376. Standard solid-phase immunoassays with monoclonal antibodies involve the formation of a complex between antibody adsorbed on a solid phase (capture antibody), antigen, and antibody to another epitope of the antigen conjugated with an enzyme (tracer antibody). Thus, a sandwich is formed: solid phase-capture antibody-antigen-tracer antibody. In the reaction catalyzed by the sandwich, the activity of the antibody-conjugated enzyme is proportional to the antigen concentration in the incubation medium. The standard sandwich method is also called double antigen bridging immunoassay because capture and tracer antibodies bind to different epitopes of the antigen.

The invention refers to methods for the assessment of the inhibitory activity of antibodies against insulin-like growth factor I receptor (IGF-1R), methods for predicting the pharmacokinetic and pharmacodynamic properties of such antibodies and methods for the prediction of a clinical outcome of a treatment course in a patient wherein such an antibody is administered. Assays for the determination of IGF-1R are well known in the state of the art (e.g. WO 02/053596, WO 2004/087756 and WO 2005/005635) however there are used as samples either IGF-1R overexpressing cells (3T3 cells) or measurement is performed by flow cytometry (Cohen, B.D., Clin. Cancer Res. 11 (2005) 2063-2073).

There is a need for measuring and monitoring the pharmacokinetic and pharmacodynamic outcome of a treatment of a patient with an antibody against IGF-1R. Such measuring and monitoring can be performed e.g. by measuring the IGF-1R expression levels (Kornprat, P., J. Clin. Path. 59 (2006) 202-206) or the IGF-1R phosphorylation status (Chen, J.W., Am. J. Physiol. Endocrinol. Metab. 284 (2003) e1149-e1155) in tumor biopsies. It is also suggested to measure IGF-1R depletion in patients (Cohen, B.D., Clin. Cancer Res. 11 (2005) 2063-2073). Ex vivo treatment of blood with an antibody against IGF-1R resulted in the down-regulation of IGF-IR on human peripheral blood mononuclear cells as monitored by flow cytometry.

### Summary of the Invention

The invention comprises a method for the determination of 1GF-1R in a sample by a solid-phase immunoassay characterized in that said sample is a preparation of lysed mammal peripheral blood lymphocytes (PBLs). Such a sample is further denoted as "lysed PBL sample" or "lysed PBLs". The mammal is preferably a human being or an ape.

It was surprisingly found, that though the range of IGF-1R expression on PBLs is between 250 and 2,300 molecules (Cohen, B.D., Clin. Cancer Res. 11 (2005) 2063-2073) it is possible to determine IGF-1R on lysed PBLs by a solid-phase immunoassay according to the invention.

The invention comprises a method according to the invention, characterized in that the IGF-1R protein level is determined in a lysed PBL sample as a measure for the in vivo activity of an antibody against IGF-1R administered to said mammal.

The invention comprises a method according to the invention, for the assessment of the in vivo activity of an antibody against IGF-1R in a mammal treated with said antibody characterized in that in a lysed PBL sample of said mammal the IGF-1R protein level is determined as a measure for the in vivo activity of said antibody.

The invention comprises a method according to the invention for the prediction of a pharmacodynamic or pharmacokinetic property of an antibody against IGF-1R in a mammal treated with said antibody characterized in that in a lysed PBL sample of said mammal the IGF-1R protein level is determined as a measure for said prediction.

The invention comprises a method according to the invention for the predicting a clinical outcome or determining a treatment course in a patient wherein an antibody against IGF-1R is administered, characterized in comprising the steps of determining the IGF-1R protein level in a lysed PBL sample of said patient at a first time, comparing said protein level with said level determined at a second time.

The invention comprises a method according to the invention, characterized in that the time for sampling is either before and during and/or after administration.

The invention comprises a method according to the invention, characterized in that the IGF-1R protein level is measured by ELISA.

The invention comprises a method according to the invention, characterized in that the first and second antibody used in the ELISA are two monoclonal antibodies binding to different epitopes or a monoclonal antibody and a polyclonal antibody against IGF-1R.

A first and a second antibody against IGF-1R can therefore be used for the manufacturing of an immunological assay for the assessment of the in vivo activity of a third antibody against IGF-1R, wherein said mammal has been treated with said third antibody antibody, characterized in that in a lysed PBL sample of said mammal the IGF-1R protein level is determined as a measure for the in vivo activity of said third antibody.

A first and a second antibody against IGF-1R can therefore be used for the manufacturing of an immunological assay for the prediction of a pharmacodynamic or pharmacokinetic property of a third antibody against IGF-1R in a mammal treated with said third antibody characterized in that in a lysed PBL sample of said mammal the IGF-1R protein level is determined as a measure for said prediction.

A first and a second antibody against IGF-1R can therefore be used for the manufacturing of an immunological assay for predicting a clinical outcome or determining a treatment course in a mammal wherein a third antibody is administered to the patient, characterized in comprising the steps of determining IGF-1R protein level in a lysed PBL sample of said mammal at a first time and comparing said protein level with said level determined at a second time.

In such use the time for sampling is either before, during and/or after administration of the third antibody.

For such use the antibodies used in the immunological assay, preferably ELISA, are two monoclonal antibodies binding to different epitopes or a monoclonal antibody and a polyclonal antibody against IGF-1R.

The invention comprises a method for the determination of IGF-1R in a lysed PBL sample of a mammal, characterized in that the IGF-1R protein level in said PBLs is determined, preferably as a measure for the in vivo activity of an antibody against IGF-1R administered to said mammal.

The invention comprises a method for the assessment of the in vivo activity of an antibody against IGF-1R in a lysed PBL sample of a mammal, said mammal has been treated with said antibody, characterized in that the IGF-1R protein level in said PBLs is determined as a measure for the in vivo activity of said antibody.

The invention comprises a method for the assessment of the in vivo activity of an antibody against IGF-1R in a mammal treated with said antibody, characterized in that in a lysed PBL sample of said treated mammal the IGF-1R protein level is determined as a measure for the in vivo activity of said antibody.

The invention further comprises a method of prediction of a pharmacodynamic or pharmacokinetic property of an antibody against IGF-1R in a mammal treated with said antibody, characterized in that in a lysed PBL sample of said mammal the IGF-1R protein level is determined as a measure for said prediction.

The invention further comprises a method of predicting a clinical outcome or determining a treatment course in a patient wherein an antibody against IGF-1R is administered, characterized in comprising the steps of determining IGF-1R protein level in a lysed PBL sample of said patient at a first time, comparing said protein level with said level determined at a second time. The time for sampling is either before, during and/or after administration.

All these methods are performed outside the mammal and the IGF-1R protein level is determined in vitro.

Preferably PBLs are isolated from the sample, preferably from buffy coat- usually by centrifugation using Ficoll gradient - before the determination methods are performed. As sample for the methods according to the invention is preferably used a preparation of 10⁶ to 10⁸ PBL cells/250 µl. The PBLs are lysed and solubilized in (e.g. PBS buffered) aqueous solution. Lysis is performed e.g.. by the use of an alkylaryl polyether alcohol like octylphenoxypolyethoxyethanol (Triton® X-100) or CHAPS (e.g. 1% (v/v)).

A first and a second antibody against IGF-1R can therefore be used for the manufacturing of an immunological assay for the assessment of the in vivo activity of a third antibody against IGF-1R wherein said mammal has been treated with said third antibody antibody, characterized in that in a lysed PBL sample of said mammal the IGF-1R protein level is determined as a measure for the in vivo activity of said third antibody.

A first and a second antibody against IGF-1R can therefore be used for the manufacturing of an immunological assay for the prediction of a pharmacodynamic or pharmacokinetic property of a third antibody against IGF-1R in a mammal treated with said third antibody characterized in that in a lysed PBL sample of said mammal the IGF-1R protein level is determined as a measure for said prediction.

A first and a second antibody against IGF-1R can therefore be used for the manufacturing of an immunological assay for predicting a clinical outcome or determining a treatment course in a mammal wherein a third antibody is administered to the patient comprising the steps of determining IGF-1R protein level in a lysed PBL sample of said mammal at a first time and comparing said protein level with said level determined at a second time. The time for sampling is either before and during and/or after administration.

The antibodies which can be used in the determination methods of the invention are preferably two monoclonal antibodies binding to different epitopes or a monoclonal antibody and a polyclonal antibody. The first antibody is immobilized and the second antibody is the detection antibody and contains a detectable label.

Antibodies which can be used for the invention bind to IGF-1R human (EC 2.7.1.112, SwissProt P08069) and preferably to the alpha-chain of the receptor. Such monoclonal antibodies useful according to the invention are e.g. described in WO 2004/087756 and WO 2005/005635. Such an antibody is also useful as third (therapeutic) antibody. Hybridoma cell lines producing useful monoclonal antibodies were deposited with Deutsche Sammlung von Mikroorganismen und Zellkulturen GmbH (DSMZ), Germany.

| **Cell line** | **Deposition No.** | **Date of deposit** |
|---|---|---|
| <IGF-1R> HUMAB-Clone 22 | DSM ACC 2594 | 09.05.2003 |
| <IGF-1R> HUMAB-Clone 18 | DSM ACC 2587 | 10.04.2003 |
| <IGF-1R> HUMAB Clone 1a | DSM ACC 2586 | 10.04.2003 |
| <IGF-1R> HUMAB Clone 23 | DSM ACC 2588 | 10.04.2003 |
| <IGF-1R> HUMAB-Clone 8 | DSM ACC 2589 | 24.04.2003 |

The antibody which can be used for the invention is further characterized by an affinity to IGF-1R of 10⁻⁸ M (K_{D}) or less, preferably of 10⁻⁹ M and more preferably of about 10⁻¹⁰ to 10⁻¹³ M.

The IGF-1R protein level is determined by an immunological assay, more preferably by ELISA, whereby one antibody is immobilized. Immobilizing is preferably performed via a biological binding pair, which is preferably hapten-antihapten (like digoxigenin-antibody against digoxigenin) or biotin-(strept)avidin. In a preferred embodiment of the invention the first antibody is biotinylated and immobilized on a streptavidin (or other avidin analog) matrix. Biotinylation or hapten-coupling is performed in such a manner that the antibody retains still an affinity to IGF-1R of 10⁻⁹ M (K_{D}) or less, preferably of about 10⁻¹⁰ to 10⁻¹³ M.

Preferably the mammal is a human patient in the need of a tumor therapy or a non-human mammal treated for in vivo animal trials.

### Detailed Description of the Invention

The term "antibody" encompasses the various forms of antibodies including, but not being limited to, whole antibodies, antibody fragments, human antibodies, humanized antibodies and genetically engineered antibodies. Preferably one of the first and second antibodies is monoclonal and the other is polyclonal from a non-human animal like rabbit or goat.

"Antibody fragments" comprise a portion of a full length antibody, generally at least the antigen binding portion or the variable region thereof which is adequate and useful for an assay according to the invention. Examples of antibody fragments include Fab and F(ab)₂ fragments.

The term "antibody against IGF-1R" or "IGF-1R antibody" as used herein refers to an antibody which binds specifically to human IGF-1R and preferably shows therefore no detectable crossreactivity with other human proteins.

The term "binding to IGF-1R" as used herein means the binding of the antibody to IGF-1R in an in vitro assay, preferably in a binding assay in which the antibody is bound to a surface and binding of IGF-1R is measured by Surface Plasmon Resonance (SPR). Binding means a binding affinity (K_{D}) of 10⁻⁸ M or less, preferably of 10⁻⁹ M or less and more preferably 10⁻¹³ to 10⁻¹⁰ M.

The term "in vivo activity of antibody against IGF-1R" as used herein refers to the well known activity known for such antibodies which is binding to IGF-1R and inhibiting ligand binding. Antibody-mediated blockade of ligand binding to the IGF-1R inhibits downstream signaling of the two major insulin-like growth factor (IGF) pathways, mitogen-activated protein kinase and phosphatidylinositol 3'-kinase/Akt. As a result, the mitogenic and proliferative potential of IGF-1 and IGF-2 were significantly reduced. In addition the antibody induces IGF-1R internalization and degradation on specific binding to tumor cells, resulting in a significant (at least 80%) reduction in cell surface presented receptor molecules. (see e.g. Mitsiades, C.S., Cancer Cell 5 (2004) 221-230; Grimberg, A., Cancer Biology & Therapy 2 (2003) 630-635).

Peripheral blood mononuclear cells/lymphocytes were isolated from buffy coats according to the state of the art, see e.g. Rees, G. and Gough R., J. Med. Lab. Tech. 25 (1968) 117-118 and Figdor, C., etal., J. Immunol. Methods 55 (1982) 221-229.

The immunological determination of an antibody against IGF-1R occurs by a double antigen bridging immunoassay comprising a capture antibody (first antibody) and a tracer antibody (second antibody) conjugated to the detectable label. The sandwich ELISA method is characterized by measuring the concentration of antigen using two kinds of monoclonal IGF-1R antibodies (the first and the second antibody) which recognize different epitopes of the antigen, alternately, with one kind of monoclonal IGF-1R antibody and one kind of polyclonal IGF-1R antibody. The procedure of this sandwich ELISA consists usually of three stages. For example, in the first stage, an antigen-containing sample is poured on a measurement plate on which the first monoclonal/polyclonal antibody has been adsorbed; IGF-1R in the sample is bound to the first antibody. In the second stage, the substances in the sample other than the antigen are washed off with a washing agent. Then, in the third stage, a solution of the second antibody, labeled with reporter molecules, such as an enzyme or radioisotope, are poured on the plate; the labeled antibody binds to IGF-1R having been bound to the first antibody and the label is detected. ELISA methods are well known in the state of the art and reviewed e.g. by Hildebrand R.L., Rapid diagnosis in infectious disease, Rytel, M.W. (ed.), Boca Raton (1979) pp. 71-88.

Preferably conjugation of an antibody to its conjugation partner is performed by chemically binding via N-terminal and/or ε-amino groups (lysine), ε-amino groups of different lysines, carboxy-, sulfhydryl-, hydroxyl- and/or phenolic functional groups of the amino acid backbone of an antibody and/or sugar alcohol groups of the carbohydrate structure of an antibody.

Preferably the first antibody is immobilized via a specific binding pair. Such a binding pair (first component/second component) is, for example, streptavidin or avidin/biotin, antibody/antigen (see, for example, Hermanson, G.T., Bioconjugate Techniques, Academic Press, Inc. (1996)), lectin/polysaccharide, steroid/steroid binding protein, hormone/hormone receptor, enzyme/substrate, IgG/Protein A and/or G, etc. Preferably, the first antibody is conjugated to biotin and immobilization is performed via immobilized avidin or streptavidin.

Alternatively the first antibody is conjugated to the solid phase by passive adsorption. Passive adsorption is, e. g., described by Butler, J.E., Solid Phases in Immunoassay, In: Immunoassay, Diamandis, E.P., and Christopoulos, T.K. (eds.), Academic Press, Inc., San Diego, California (1996), pp. 205-225.

In a preferred embodiment of the invention, the second antibody is conjugated to a detectable label, preferably conjugated via a specific binding pair. Such a binding pair (first component/second component) is, for example, streptavidin or avidin/biotin, antibody/antigen (see, for example, Hermanson, G.T., Bioconjugate Techniques, Academic Press, Inc., San Diego, California (1996), lectin/polysaccharide, steroid/steroid binding protein, hormone/hormone receptor, enzyme/substrate, IgG/Protein A and/or G, etc. Preferably, the second antibody is conjugated via digoxigenin and an antibody against digoxigenin to the detectable label. Alternatively the second antibody is conjugated to an electrochemiluminescent label, like a ruthenium bispyridyl complex.

Monoclonal antibodies contain as proteins a number of reactive side chains. Such reactive chemical groups of antibodies are, for example, amino groups (lysines, alpha-amino groups), thiol groups (cystines, cysteine, and methionine), carboxylic acid groups (aspartic acid, glutamic acid) and sugar-alcoholic groups.

Solid supports for the immunoassays according to the invention are widely described in the state of the art (see, e.g., Butler, J.E., Methods 22 (2000) 4-23).

The principles of different immunoassays are described, for example, by Hage, D.S., in Anal. Chem. 71 (1999) 294R-304R. Lu, B., et al., in Analyst. 121 (1996) 29R-32R, report the orientated immobilization of antibodies for the use in immunoassays. Avidin-biotin-mediated immunoassays are reported, for example, by Wilchek, M., and Bayer, E.A., Methods Enzymol. 184 (1990) 467-469.

Monoclonal antibodies and their constant domains contain as proteins a number of reactive side chains for coupling to a binding partner like a surface, a protein, a polymer, such as PEG, cellulose or polystyrol, an enzyme or a member of a binding pair. Chemical reactive groups of antibodies are, for example, amino groups (lysines, alpha-amino groups), thiol groups (cystines, cysteines, and methionines), carboxylic acid groups (aspartic acids, glutamic acids) and sugar-alcoholic groups. Such methods are e.g. described by Aslam, M. and Dent, A., Bioconjuation, MacMillan Reference Ltd. (1999), pp. 50-100.

The term "solid phase" means a non-fluid substance, and includes particles (including microparticles and beads) made from materials such as polymer, metal (paramagnetic, ferromagnetic particles), glass, and ceramic; gel substances such as silica, alumina, and polymer gels; capillaries, which may be made of polymer, metal, glass, and/or ceramic; zeolites and other porous substances; electrodes; microtiter plates; solid strips; and cuvettes, tubes or other spectrometer sample containers. A solid phase component of an assay is distinguished from inert solid surfaces with which the assay may be in contact in that a "solid phase" contains at least one moiety on its surface, which is intended to interact with the second antibody. A solid phase may be a stationary component, such as a tube, strip, cuvette or microtiter plate, or may be non-stationary components, such as beads and microparticles. Microparticles can also be used as a solid phase for homogeneous assay formats. A variety of microparticles that allow either non-covalent or covalent attachment of proteins and other substances may be used. Such particles include polymer particles such as polystyrene and poly(methylmethacrylate); gold particles such as gold nanoparticles and gold colloids; and ceramic particles such as silica, glass, and metal oxide particles. See for example Martin, C.R., et al., Anal. Chem. 70 (1998) 322A-327A. Chromogens (fluorescent or luminescent groups and dyes), enzymes, NMR-active groups or metal particles, haptens, e.g. digoxigenin, are examples of detectable labels. The detectable label can also be a photoactivatable crosslinking group, e.g. an azido or an azirine group. Metal chelates which can be detected by electrochemoluminescence are also preferred signal-emitting groups, with particular preference being given to ruthenium chelates, e.g. a ruthenium (bispyridyl)₃²⁺ chelate. Suitable ruthenium labeling groups are described, for example, in EP 0 580 979, WO 90/05301, WO 90/11511 and WO 92/14138.

The following examples and figures are provided to aid the understanding of the present invention, the true scope of which is set forth in the appended claims.

### Description of the Figures

- **Figure 1**: The levels of the same samples were measured in five independent experiments, labeled from 1 to 5. Same colors indicate identical samples. Standard deviations ranged between 11 and 14%.
- **Figure 2**: Fresh isolated peripheral leukocytes from Cynomolgus (Start Population) were tested for their IGF1-R concentration and compared to an overnight culture of the cells (untreated) and an overnight culture of the cells treated with antibody 18 (treated). On the left panel arbitrary units per mg of protein are plotted.

### Example 1

### PBL isolation and lysis

### a) Isolation of human PBLs

200 µl of Liquemine (Roche Diagnostics GmbH, DE) were added to 160 ml of RPMI/Hepes (10mM Hepes Buffer in RPMI). The peripheral blood cells ("Buffy coats") from 0.51 of blood were resuspended in this solution. The separation of the blood cell types was carried out in 50 ml GREINER Leucosep tubes (Article No. 7.227.290). Six tubes were used for the isolation of PBLs from one buffy coat. To prepare the tubes, 15 ml of Lymphocyte Selection Medium (LSM, ICN No. 50494) were transferred into each tube and the tubes were centrifuged at 1000 x g for 30 seconds. 39 ml of the blood suspension were layered on top of the LSM solution and centrifuged at 1000 x g for 10 min at 18°C. The PBLs appear as a white band directly above the septum and were recovered with a 10 ml pipette and transferred to four 50 ml tubes. The tubes were then filled to 50 ml with RPMI/Hepes and centrifuged at 250 x g for 10 min at 18°C. The supernatant was discarded and the cells resuspended in a small volume of RPMI/Hepes. All cells were combined in a single 50 ml tube and washed again in RPMI/Hepes. The supernatant was discarded and the cells were resuspended in 10 ml of pre-warmed ALT for lysis of the contaminating erythrocytes. ALT was prepared by combining 300 ml of solution A (0.16 M NH₄Cl) with 100 ml of Solution B (0.18 M Tris-HCl, pH 7.65), adjusting the pH to 7.2 and sterilizing the solution by filtration (0.22 µm). After incubation of the Cell suspension in ALT for 3 min at 37°C, the tube was filled up to 50 ml with RPMI/Hepes and the cells were centrifuged at 250 x g for 5 min. Finally, the cells were washed again twice in RPMI/Hepes and resuspended in 50 ml of RPMI/Hepes. Finally, the cells were counted. Alternatively to the freshly prepared cells, frozen PBLs were also used in some instances. For freezing, cells were resuspended in freeze medium (10% DMSO in heat-inactivated human serum) at a density of 10 million cells/ml. The suspension is slowly cooled down to -80°C. For thawing, cells are quickly thawed and then diluted slowly without stirring by stepwise addition of medium.

### b) Isolation of Cynomolgus PBLs

Blood (24 ml, 3 ml each from 8 different monkeys) was collected in vacutainers containing heparin. 56 ml of PBS was added and the suspension layered on top of a cushion of LSM (ICN No. 50494). The tubes were spun at 1000 x g for 10 min. The PBLs were removed, washed twice in PBS and counted.

### c) PBL treatment

Between two and 10 million cells per well were seeded in 2 ml of medium (RPMI with 10% FCS) in 6-well cell culture plates. Controls were left untreated. To half of the wells, antibody against IGF-1R (2.0 mg/ml) was added to an end concentration of 100 nM. The cells were incubated overnight at 37°C and with 5% CO₂. As a control, an aliquot of cells was centrifuged and frozen before the cell culture step for further analysis.

### d) PBL Lysis

The cells were harvested by centrifugation and washed twice in PBS. The pellets were frozen. Shortly before analysis, the cell pellets were thawed and resuspended in 200 µl of lysis buffer (50 mM Tris-HCl, pH 7.5, 1 mM EDTA, 1 mM EGTA, 100 mM NaF, 1% Triton-X-100, 20% glycerol) vortexed, put into an ultrasonic water bath for 2 min and centrifuged.

### Example 2

### ELISA Assay

The IGF-1 Receptor in peripheral blood lymphocytes is captured by a biotinylated antibody against the α-domain of the receptor on a streptavidin coated microplate (SA-MTP) (antibody 18). Bound IGF-1R is detected by a rabbit polyclonal IGF-1R antibody and an anti-rabbit-HRP coupled antibody.

The IGF-1R level was measured as IGF-1R protein amount relatively to total protein amount of the cell. Cell lysates were produced, adjusted to the same protein concentration and measured in the ELISA assay. In the linear range of the assay the absorption signal at 450 nm corresponds to the relative amount of IGF-1R level.

### Materials:

- 96-Well streptavidin coated polystyrene plates (Nunc)
- PBST: 10xPBS-Buffer Solution diluted 1:10 with H₂O + 0,2% Tween20
- BSA
- PAK<IGF-1Rβ>Ra-C20-IgG (Santa Cruz Biotechnology, #sc-713)
- PAK<Rabbit>G-IgG-HRP (Cell Signaling #7074)
- 3,3'-5,5'-tetramethylbenzidine
- 1M H₂SO₄
- biotinylated MAK<IGF-1Rα>Hu-AK1α-IgG (DSM ACC 2586)

### Plate coating:

- dilute biotinylated antibody 1:200 in 3%BSA/PBST
- add 100 µl/well to SA-MTP
- incubate for 1h at room temperature on a shaker
- wash with 200 µl PBST/well

### Sample preparation:

- After washing with PBS, peripheral blood lymphocytes (PBLs, 1x10⁷ cells) are reisolated by centrifugation and the cell pellets are solubilized in 250 µl of lysis buffer. To ensure the IGF-1R concentrations of the lysates to be in the linear range of the ELISA assay, a dilution series is be performed.

### Assay procedure:

- add 100 µl/well sample to assay plate
- incubate for 1h on room temperature
- wash with 200 µl PBST/well
- add 100 µl/well PAK<IGF-1Rβ>Ra-C20-IgG (Santa Cruz Biotechnology, #sc-713) 1:1000 in 3% BSA/PBST to the plate
- incubate for 1h at room temperature
- wash with 200 µl PBST/well
- add 100 µl/well PAK<Rabbit>G-IgG-HRP 1:5000 in 3% BSA/PBST to the plate
- incubate for 1h at room temperature
- wash with 200 µl PBST/well
- add 100 µl/well 3,3'-5,5'-tetramethylbenzidine to assay plate
- incubate for 0,5h at room temperature
- stop the reaction with 25µl/well 1M H₂SO₄
- measure absorption at 450 nm

In the absence of pure recombinant protein, the ELISA signal obtained with a dilution of 1 : 100 of the positive control was defined as arbitrary unit "U". Different dilutions of the sample were measured and the means of at least two values were calculated.

### Determination of the changes in IGF-1R levels in PBL cultures treated with antibody against IGF-1R:

PBLs from 20 donors were cultured in the presence and absence of antibody 18. Nine of the samples were from frozen stocks, eleven were determined with freshly prepared cells. Down-regulation of IGF-1R levels after 24 h of treatment with antibody against IGF-1R is determined. In all cell cultures, the levels of IGF-1R were reduced in the antibody treated compared to the untreated cells. The levels of IGF-1R after treatment ranged between 0 and 62% of the levels detected in the control cultures. Therefore, down-regulation of the receptor appears a general phenomenon after down-regulation of the signaling cascade by the therapeutic antibody. The baseline levels of receptor expression varied strongly between donors, so that it appears likely that "threshold" levels cannot be established and a time course of the levels will have to be determined throughout the treatment period for each patient, including values before treatment as baseline controls. For 19/20 donors, the levels before cell culture were lower than the levels after cell culture (without treatment). For 17/20 donors however, these levels were still higher than those measured in the cell cultures after treatment with the therapeutic antibody.

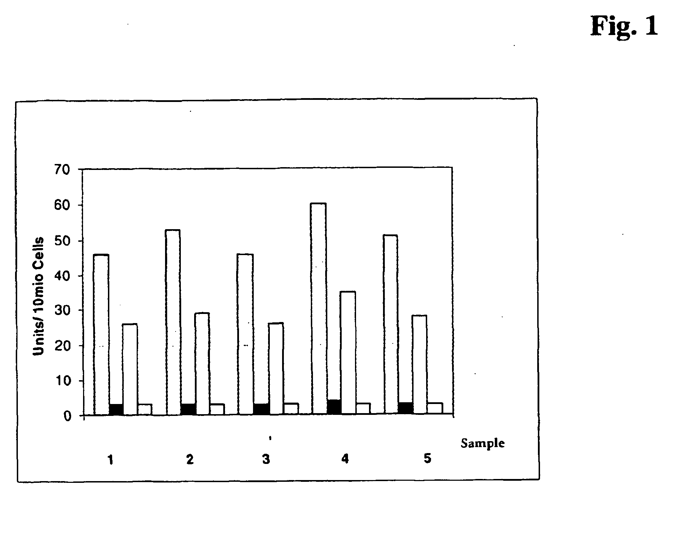

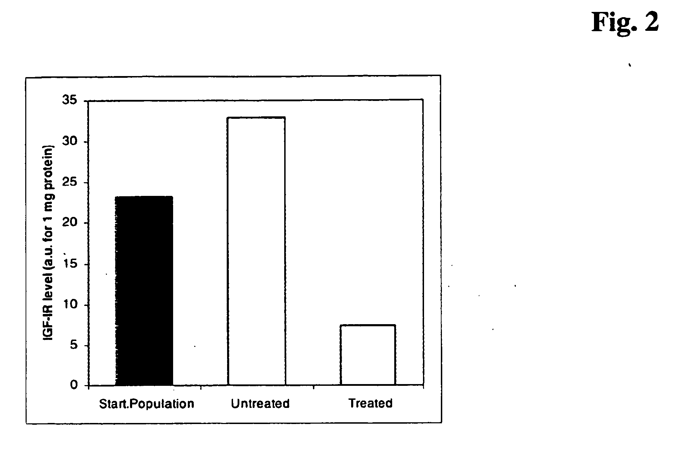

For two cell preparations, the reduction of IGF-1R levels was tested with different concentrations of huMab IGF-1R. Even lower levels of IGF-1R were detected at lower concentrations (Fig. 1 and 2).

In order to confirm that the assay can be applied in Cynomolgus monkeys in pre-clinical experiments, an incubation of monkey PBLs with and without therapeutic antibody was performed. As in the human samples, down-regulation of the IGF-1R concentration was detected in cynomolgus lymphocytes. The rates of down-regulation of receptor levels were comparable to those detected with human material.

## Claims

1. Method for the determination of IGF-1R in a sample by a solid-phase immunoassay **characterized in that** said sample is a preparation of lysed mammal peripheral blood lymphocytes (lysed PBL sample).

2. Method according to claim 1, **characterized in that** the mammal is a human being or an ape.

3. Method according to claim 1 or 2, **characterized in that** the IGF-1R protein level is determined as a measure for the in vivo activity of an antibody against IGF-1R administered to said mammal.

4. Method for the assessment of the in vivo activity of an antibody against IGF-1R in a mammal treated with said antibody, **characterized in that** in a lysed PBL sample of said mammal the IGF-1R protein level is determined as a measure for the in vivo activity of said antibody.

5. Method of prediction a pharmacodynamic or pharmacokinetic property of an antibody against IGF-1R in a mammal treated with said antibody, **characterized in that** in a lysed PBL sample of said mammal the IGF-1R protein level is determined as a measure for said prediction.

6. Method of predicting a clinical outcome or determining a treatment course in a patient wherein an antibody against IGF-1R has been administered, **characterized in** comprising the steps of determining the IGF-1R protein level in a lysed PBL sample of said patient at a first time, comparing said protein level with said level determined at a second time.

7. Method according to claim 6, **characterized in that** the time for sampling is either before, during and/or after administration.

8. Method according to claims 1 to 7, **characterized in that** the IGF-1R protein level is measured by ELISA.

9. Method according to claims 1 to 8, **characterized in that** first and second antibody used are two monoclonal antibodies binding to different epitopes or a monoclonal antibody and a polyclonal antibody against IGF-1R.

## Patentansprüche

1. Verfahren zur Bestimmung von IGF-1 R in einer Probe mittels eines Festphasenimmunoassays, **dadurch gekennzeichnet, dass** die Probe eine Präparation von lysierten peripheren Blutlymphozyten (lysierte PBL-Probe) von einem Säugetier ist.

2. Verfahren nach Anspruch 1, **dadurch gekennzeichnet, dass** das Säugetier ein Mensch oder ein Affe ist.

3. Verfahren nach Anspruch 1 oder 2, **dadurch gekennzeichnet, dass** der IGF-1R-Proteinspiegel als Maß für die in vivo-Aktivität eines dem Säugetier verabreichten Antikörpers gegen IGF-1R bestimmt wird.

4. Verfahren zur Feststellung der in vivo-Aktivität eines Antikörpers gegen IGF-1R in einem mit dem Antikörper behandelten Säugetier, **dadurch gekennzeichnet, dass** in einer lysierten PBL-Probe des Säugetiers der IGF-1 R-Proteinspiegel als Maß für die in vivo-Aktivität des Antikörpers bestimmt wird.

5. Verfahren zur Vorhersage einer pharmakodynamischen oder pharmakokinetischen Eigenschaft eines Antikörpers gegen IGF-1R in einem mit dem Antikörper behandelten Säugetier, **dadurch gekennzeichnet, dass** in einer lysierten PBL-Probe des Säugetiers der IGF-1 R-Proteinspiegel als Maß für die Vorhersage bestimmt wird.

6. Verfahren zur Vorhersage eines klinischen Ergebnisses oder zur Bestimmung eines Behandlungsverlaufs in einem Patienten, wobei ein Antikörper gegen IGF-1 R verabreicht wurde, **dadurch gekennzeichnet, dass** es die Schritte Bestimmen des IGF-1 R-Proteinspiegels in einer lysierten PBL-Probe des Patienten zu einem ersten Zeitpunkt, Vergleichen des Proteinspiegels mit dem zu einem zweiten Zeitpunkt bestimmten Spiegel umfasst.

7. Verfahren nach Anspruch 6, **dadurch gekennzeichnet, dass** der Zeitpunkt der Probenentnahme entweder vor, während und/oder nach der Verabreichung ist.

8. Verfahren nach einem der Ansprüche 1 bis 7, **dadurch gekennzeichnet, dass** der IGF-1R-Proteinspiegel mittels ELISA gemessen wird.

9. Verfahren nach einem der Ansprüche 1 bis 8, **dadurch gekennzeichnet, dass** der erste und zweite verwendete Antikörper zwei monoklonale Antikörper, die an unterschiedliche Epitope binden, oder ein monoklonaler Antikörper und ein polyklonaler Antikörper gegen IGF-1R sind.

## Revendications

1. Procédé de détermination de l'IGF-1R dans un échantillon par analyse immunologique en phase solide, **caractérisé en ce que** ledit échantillon est une préparation de lymphocytes de sang périphérique de mammifère lysés (échantillon de LSP lysés).

2. Procédé selon la revendication 1, **caractérisé en ce que** le mammifère est un être humain ou un grand singe.

3. Procédé selon la revendication 1 ou 2, **caractérisé en ce que** le niveau de protéine IGF-1R est déterminé en tant que mesure de l'activité *in vivo* d'un anticorps dirigé contre l'IGF-1R administré audit mammifère.

4. Procédé d'évaluation de l'activité *in vivo* d'un anticorps dirigé contre l'IGF-1R chez un mammifère traité avec ledit anticorps, **caractérisé en ce que** l'on détermine le niveau de protéine IGF-1R dans un échantillon de LSP lysés dudit mammifère en tant que mesure de l'activité *in vivo* dudit anticorps.

5. Procédé de prédiction d'une propriété pharmacodynamique ou pharmacocinétique d'un anticorps dirigé contre l'IGF-1R chez un mammifère traité avec ledit anticorps, **caractérisé en ce que** l'on détermine le niveau de protéine IGF-1R dans un échantillon de LSP lysés dudit mammifère en tant que mesure pour ladite prédiction.

6. Procédé de prédiction d'un résultat clinique ou de détermination de l'évolution d'un traitement chez un patient auquel a été administré un anticorps dirigé contre l'IGF-1R, **caractérisé en ce qu'**il comprend les étapes de détermination du niveau de protéine IGF-1R dans un échantillon de LSP lysés dudit patient à un premier moment, et la comparaison dudit niveau de protéine avec ledit niveau déterminé à un deuxième moment.

7. Procédé selon la revendication 6, **caractérisé en ce que** le moment du prélèvement de l'échantillon se situe avant, pendant et/ou après l'administration.

8. Procédé selon les revendications 1 à 7, **caractérisé en ce que** le niveau de protéine IGF-1R est déterminé par une analyse de type ELISA.

9. Procédé selon les revendications 1 à 8, **caractérisé en ce que** le premier et le deuxième anticorps utilisés sont deux anticorps monoclonaux se liant à des épitopes différents ou un anticorps monoclonal et un anticorps polyclonal dirigés contre l'IGF-1R.
